# EUROPÄISCHE PATENTANMELDUNG

(11) **EP 0 571 783 A2**
(43) Veröffentlichungstag der Anmeldung: **01.12.1993**
(21) Anmeldenummer: 93107127.8
(22) Anmeldetag: 03.05.1993
(51) Int. Cl.: G01N 33/00, F23N 5/00

(54) **Verfahren zur Kalibrierung von Gassensoren in Messgeräten für die Gasanalyse, Rauchgasanalyse und/oder die Wirkungsgradermittlung an Feuerungen sowie Messgerät zur Durchführung dieses Verfahrens**

(30) Priorität: 29.05.1992 DE 4217893
(71) Anmelder: MSI ELEKTRONIK GMBH, W-5800 Hagen 1 (DE)
(72) Erfinder: Oppermann, Werner, W-5750 Menden 1 (DE)
(74) Vertreter: Patentanwälte Meinke, Dabringhaus und Partner

(57) **Zusammenfassung**

Mit einem Verfahren zur Kalibrierung von Gassensoren in Meßgeräten für die Gasanalyse, Rauchgasanalyse und/oder die Wirkungsgradermittlung an Feuerungen, wobei das Meßsignal des jeweiligen Gassensors, welches mit der Konzentration eines Gasbestandteiles des zu messenden Gases variiert, einer Recheneinrichtung zugeleitet wird, soll eine Lösung geschaffen werden, mit der bei derartigen Meßvorgängen eine selbsttätige Kalibrierung des Referenzwertes für den jeweils zu messenden Gasbestandteil ohne vorherige zeitaufwendige Vergleichsmessung ermöglicht wird.

Dies wird dadurch erreicht, daß während des eingeschalteten Zustands des Meßgerätes kontinuierlich die Konzentration des Gasbestandteiles gemessen und bei Vorliegen eines stabilen Meßsignales im Bereich des üblichen Gasreferenzwertes automatisch von der Recheneinrichtung eines Meßwertkalibrierung durchgeführt wird.

## Beschreibung

Die Erfindung betrifft ein Verfahren zur Kalibrierung von Gassensoren in Meßgeräten für die Gasanalyse, Rauchgasanalyse und/oder die Wirkungsgradermittlung an Feuerungen, wobei das Meßsignal des jeweiligen Gassensors, welches mit der Konzentration eines Gasbestandteiles des zu messenden Gases variiert, einer Recheneinrichtung zugeleitet wird, sowie ein Meßgerät für die Gasanalyse, Rauchgasanalyse und/oder die Wirkungsgradermittlung an Feuerungen zur Durchführung dieses Verfahrens mit Gassensoren und einer mit diesem verbundenen Recheneinrichtung zur Auswertung der Meßsignale.

Zur Messung von Gasbestandteilen im Rauchgas von z.B. Heizungsanlagen von Haushalten ist es bekannt, mobile Meßgeräte einzusetzen, die mit Gassensoren ausgerüstet sind, welche in den Kamin oder dgl. eingeführt werden. Bei derartigen Gassensoren besteht jedoch das Problem, daß diese Sensoren zur Drift neigen, d.h. der einmal eingestellte Referenzwert (z.B. Luft mit 20,9 % O₂) verstellt sich selbsttätig, so daß eine Driftkorrektur durchzuführen ist, um genaue Meßwerte erhalten zu können.

Hierzu ist es beispielsweise aus der DE 30 42 670 C2 bekannt geworden, nach dem Einschalten des Meßgerätes eine sogenannte Luft-Kalibrierung durchzuführen. Hierbei wird angenommen, daß die Umgebungsluft 20,9 % O₂ enthält. Nach dem Stabilisieren der Sensorsignale wird das aktuelle Sensorsignal für die Festlegung des entsprechenden Referenzpunktes genutzt. Die Stabilisierungszeit beträgt dabei zwischen 30 bis etwa 180 sec., d.h. bevor die eigentliche Messung vorgenommen werden kann, muß zunächst jeweils eine Vergleichsmessung vorgenommen werden. Da die eigentliche Messung anschließend ebenfalls etwa nur denselben Zeitraum benötigt, wird durch diesen Eichvorgang eine relativ lange Zeitspanne benötigt, in der das Meßgerät nicht zur Verfügung steht.

Aufgabe der Erfindung ist die Schaffung einer Lösung, mit der bei derartigen Meßvorgängen eine selbsttätige Kalibrierung des Referenzwertes für den jeweils zu messenden Gasbestandteil ohne vorherige zeitaufwendige Vergleichsmessung ermöglicht wird.

Diese Aufgabe wird mit einem Verfahren der eingangs bezeichneten Art erfindungsgemäß dadurch gelöst, daß während des eingeschalteten Zustands des Meßgerätes kontinuierlich die Konzentration des Gasbestandteiles gemessen und bei Vorliegen eines stabilen Meßsignales im Bereich des üblichen Gasreferenzwertes automatisch von der Recheneinrichtung eine Meßwertkalibrierung durchgeführt wird.

Mit diesem erfindungsgemäßen Verfahren ist eine automatische Driftkorrektur von Gassensoren und derartigen Meßgeräten möglich. Während das Meßgerät eingeschaltet ist, wird dazu ständig der entsprechende Gasbestandteil gemessen und der Referenzwert (z.B. Luft mit 20,9 % O₂) überwacht. Wird dabei ein stabiles Gasmeßsignal im Bereich des Referenzpunktes (z.B. Luft mit 20 bis 22 % O₂) gemessen, so bedeutet dies, daß eigentlich der Referenzwert O₂ in Luft erreicht worden ist, so daß dann der so erkannte Referenzwert für die Korrektur des Kalibrierwertes herangezogen wird. Da während der Meßvorgänge dem Meßgerät zwangsläufig immer auch Luft zugeführt wird, ist es nicht erforderlich, vor dem eigentlichen Meßvorgang eine zeitaufwendige Vergleichsmessung durchzuführen.

Zur Erhöhung der Genauigkeit des Verfahrens ist vorteilhaft vorgesehen, daß von der Recheneinrichtung die Zeitintervalle zwischen aufeinander folgenden Kalibrierwertkorrekturen überwacht und bei Überschreiten einer vorgebbaren Zeit ein Warnsignal abgegeben wird. Nimmt das Meßgerät dann innerhalb eines vorgegebenen Zeitraumes, z.B. mehrere Stunden oder Tage, keine automatische Kalibrierung vor, so bedeutet dies, daß dem Gerät keine reine Luft zugeführt worden ist. Es wird dann ein Warnsignal abgegeben, um den Benutzer dazu aufzufordern, dem Gerät reine Luft zuzuführen.

Zur Lösung der eingangs gestellten Aufgabe sieht die Erfindung auch ein Meßgerät für die Gasanalyse, Rauchgasanalyse und/oder die Wirkungsgradermittlung an Feuerungen zur Durchführung des vorbeschriebenen Verfahrens mit Gassensoren und einer mit diesen verbundenen Recheneinrichtung zur Auswertung der Meßsignale vor, welches sich dadurch auszeichnet, daß zum Kalibrieren des Meßgerätes hinsichtlich des Gassensormeßwertes der Recheneinrichtung im eingeschalteten Zustand des Meßgerätes kontinuierlich ein Gasmeßwert zugeführt ist, wobei die Recheneinrichtung zur automatischen Meßwertkalibrierung bei Vorliegen eines stabilen Meßwertes im Bereich des üblichen Referenzwertes betreibbar ist.

Mit diesem Meßgerät ist es somit auf besonders einfache Weise möglich, eine automatische Kalibrierung der Gassensoren vorzunehmen, ohne daß dazu eine zeitaufwendige Vergleichsmessung vor dem eigentlichen Meßvorgang erforderlich wäre.

In vorteilhafter Ausgestaltung sieht die Erfindung vor, daß die Recheneinrichtung mit einer Zeitmeß- und Warneinrichtung versehen ist. Dabei dient die Zeitmeßeinrichtung dazu, die Zeitintervalle zwischen einzelnen Kalibriervorgängen zu registrieren und die Warneinrichtung ist dazu vorgesehen, bei Überschreiten eines vorgegebenen Zeitraumes zwischen zwei Kalibrierintervallen ein Warnsignal abzugeben, um den Benutzer anzuzeigen, daß dem Gerät reine Luft bzw. ein anderes Gas zugeführt werden muß.

Die Erfindung ist nachstehend anhand der Zeichnung beispielsweise näher erläutert. Diese zeigt in
- Fig. 1: in einem Diagramm das typische Verhalten eines Gassensors mit Drift des Referenzwertes und in
- Fig. 2: ebenfalls in einem Diagramm das Verhalten des Gasmeßwertes mit erfindungsgemäßer Kalibrierung.

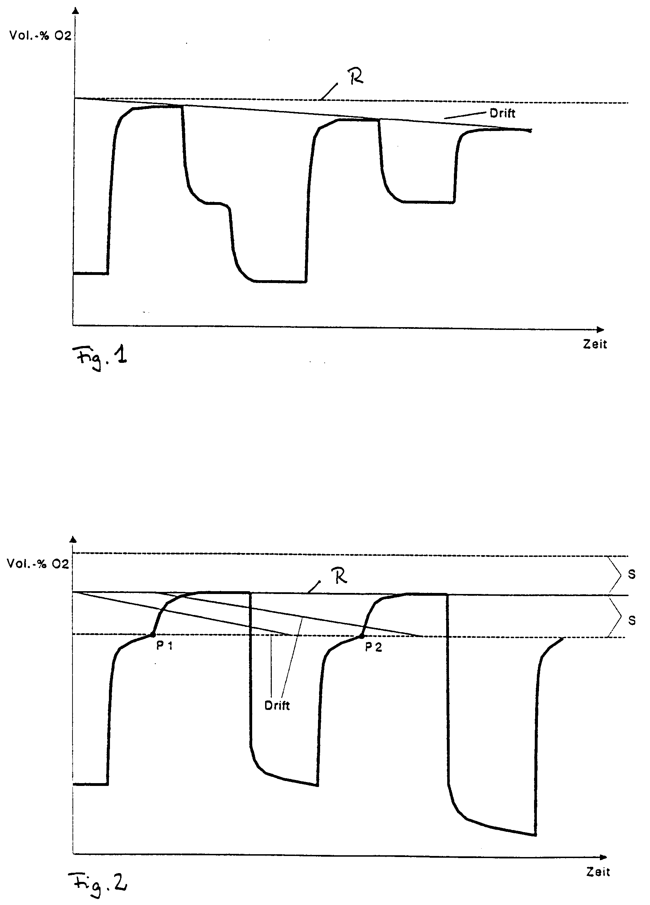

In Fig. 1 ist beispielhaft das von einem Gassensor ohne Driftkorrektur gelieferte Meßsignal für O₂ bei einem typischen Meßvorgang, z.B. der Messung an einem Rauchgaskamin einer Heizungsanlage dargestellt. Nach dem ersten Meßwert und der Entnahme des Meßgerätes aus dem Kamin wird bereits der mit R bezeichnete Referenzwert von Sauerstoff in Luft (20,9 %) nicht mehr erreicht, vielmehr wird nur ein geringerer Wert erreicht. Bei mehrmaligen Messungen weicht dieser Wert weiter nach unten ab, die Verbindungsgraden dieser Maximalwerte ist mit der Bezeichnung Drift im Diagramm nach Fig. 1 versehen.

Würde keine Driftkorrektur vorgenommen, so wären sämtliche Meßwerte, die auf den dann falsch eingestellten Referenzwert bezogen sind, nicht mehr korrekt.

Nach dem erfindungsgemäßen Verfahren wird der Referenzwert (z.B. Luft mit 20,9 % O₂) überwacht und nachgestellt, wenn eine Drift erkannt wurde.

Die üblichen elektronischen Verfahren ermitteln zeitdiskrete Meßwerte. Die zeitliche Änderung des Meßsignals wird beobachtet. Zu diesem Zweck wird laufend eine Folge von Meßwerten begutachtet. Ist die zeitliche Änderung dieses Meßwertes klein genug, so liegt ein stabiles Signal vor. Der Gassensor befindet sich dann in einem eingeschwungenen Zustand. Durch die Summierung der Abweichungen wird verhindert, daß Störungen oder Rauschen eine Steigung vortäuschen.

Unter Berücksichtigung der typischen Meßbedingungen läßt sich ein Bereich um den Referenzpunkt R definieren, der in Fig. 2 dargestellt ist. Dieser Bereich s beiderseits des Referenzpunktes R beträgt im in Fig. 2 dargestellten Beispiel beispielsweise 1 %, d.h. ein stabiler Meßwert im Bereich von 19,9 bis 21.9 % O₂ wird von der Recheneinrichtung des Meßgerätes so gedeutet, daß dieses stabile Meßsignal nur durch das reine Referenzgas (Luft) erzeugt werden kann.

Der so ermittelte stabile Meßwert P1 oder P2 (Fig. 2) entspricht dann somit nicht dem angzeigten und durch die Drift des Gassensors hervorgerufenen Wert R-s, sondern dem wahren Referenzwert R. Die Recheneinrichtung stellt dann selbsttätig den Referenzwert des Gassensors auf den wahren Referenzwert R nach, d.h. es wird eine Korrektur des Kalibrierwertes durchgeführt.

Da während der Betriebsbereitschaft des Meßgerätes ständig der betreffende Gasbestandteil gemessen wird und da zwangsläufig während der einzelnen Meßvorgänge das Meßgerät mit reiner Luft in Kontakt kommt, erfolgt kontinuierlich eine Driftkorrektur. Wird über eine bestimmte Betriebszeit des Gerätes keine Korrektur des Kalibrierwertes vorgenommen (Gerät enthält kein Referenzgas) wird der Bediener durch das Meßgerät aufgefordert, Referenzgas dem Gerät zuzuführen. Hierzu ist die Recheneinrichtung mit einer entsprechenden Zeitmeß- und Warneinrichtung versehen.

Natürlich ist die Erfindung nicht auf das dargestellte Ausführungsbeispiel beschränkt. Weitere Ausgestaltungen der Erfindung sind möglich, ohne den Grundgedanken zu verlassen. So kann für Sensoren für diverse andere Gaskomponenten, die in normaler Umgebungsluft nicht vorkommen, das Verfahren entsprechend abgewandelt werden. Dabei wird der Nullpunkt (Referenzwert) auf dieselbe Art und Weise überwacht.

## Patentansprüche

1. Verfahren zur Kalibrierung von Gassensoren in Meßgeräten für die Gasanalyse, Rauchgasanalyse und/oder die Wirkungsgradermittlung an Feuerungen, wobei das Meßsignal des jeweiligen Gassensors, welches mit der Konzentration eines Gasbestandteiles des zu messenden Gases variiert, einer Recheneinrichtung zugeleitet wird,
dadurch gekennzeichnet,
daß während des eingeschalteten Zustands des Meßgerätes kontinuierlich die Konzentration des Gasbestandteils gemessen und bei Vorliegen eines stabilen Meßsignales im Bereich des üblichen Gasreferenzwertes automatisch von der Recheneinrichtung eine Meßwertkalibrierung durchgeführt wird.

2. Verfahren nach Anspruch 1,
dadurch gekennzeichnet,
daß von der Recheneinrichtung die Zeitintervalle zwischen aufeinanderfolgenden Kalibrierwertkorrekturen überwacht und bei Überschreiten einer vorgebbaren Zeit ein Warnsignal abgegeben wird.

3. Meßgerät für die Gasanalyse, Rauchgasanalyse und/oder die Wirkungsgradermittlung an Feuerungen zur Durchführung des Verfahrens nach Anspruch 1 oder 2 mit Gassensoren und einer mit diesen verbundenen Recheneinrichtung zur Auswertung der Meßsignale,
dadurch gekennzeichnet,
daß zum Kalibrieren des Meßgerätes hinsichtlich des Gassensormeßwertes der Recheneinrichtung im eingeschalteten Zustand des Meßgerätes kontinuierlich ein Gasmeßwert zugeführt ist, wobei die Recheneinrichtung zur automatischen Meßwertkalibrierung bei Vorliegen eines stabilen Meßwertes im Bereich des üblichen Referenzwertes betreibbar ist.

4. Meßgerät nach Anspruch 3,
dadurch gekennzeichnet,
daß die Recheneinrichtung mit einer Zeitmeß- und Warneinrichtung versehen ist.
